Europäisches Patentamt

European Patent Office

Office européen des brevets

(11) Publication number: **0 361 956**
**A2**

## EUROPEAN PATENT APPLICATION

(21) Application number: 89309969.7

(22) Date of filing: 29.09.89

(51) Int. Cl.⁵: **C12N 15/11 , C12N 15/16 ,**
**C12N 15/17 , C12N 15/63 ,**
**C12N 1/21 , //C12N15/67**

(30) Priority: 30.09.88 US 252219

(43) Date of publication of application:
**04.04.90 Bulletin 90/14**

(84) Designated Contracting States:
**BE CH DE ES FR GB GR IT LI NL SE**

(71) Applicant: **ELI LILLY AND COMPANY**
**Lilly Corporate Center**
**Indianapolis Indiana 46285(US)**

(72) Inventor: **Hsiung, Hansen M.**
**4760 Cole Porter Lane**
**Indianapolis Indiana 46032(US)**

(74) Representative: **Hudson, Christopher Mark et**
**al**
**Erl Wood Manor**
**Windlesham Surrey GU20 6PH(GB)**

(54) **Increased expression of small molecular weight recombinant proteins.**

(57) The present invention discloses novel DNA sequences encoding protein derivatives that allow for high expression of small molecular weight recombinant proteins. Once the desired protein derivatives are obtained, the extra amino acid sequence can be cleaved to yield native protein. The sequence encoding methionine-x-x´-protein, wherein x and x´ are selected from the group consisting of phenylalanine, leucine, isoleucine, methionine, valine, serine, proline, threonine, alanine, tyrosine, histidine, glutamine, aparagine, lysine, apartic acid, glutaminc acid cysteine, tryptophane, arginine and glycine.

EP 0 361 956 A2

## INCREASED EXPRESSION OF SMALL MOLECULAR WEIGHT RECOMBINANT PROTEINS

The present invention discloses novel DNA sequences encoding protein derivatives that allow for increased expression of small molecular weight recombinant proteins. With the advent of recombinant DNA technology, the ability to amplify the amount of a specific protein produced by an organism has correspondingly led to increased expression of desired proteins. When proteins have small molecular weights, however, high expression is difficult to obtain. Generally accepted explanations for low expression of small molecular weight proteins include the explanation that these proteins are highly unstable and tend to be rapidly degraded by the host cell. Another explanation is that the transcriptional or translational efficiency of the underlying coding sequence is impaired.

The derivatives of the present invention are capable of high level expression and are of the formula methionine-x-tryptophane-protein and methionine-x-x´-protein. The x and x´ are representative of any one of the twenty amino acids:

Methionine (Met)
Phenylalanine (Phe)
Isoleucine (Ile)
Valine (Val)
Serine (Ser)
Proline (Pro)
Threonine (Thr)
Alanine (Ala)
Tyrosine (Tyr)
Histidine (His)
Glutamine (Gln)
Asparagine (Asn)
Lysine (Lys)
Aspartic Acid (Asp)
Glutamic Acid (Glu)
Cysteine (Cys)
Tryptophane (Trp)
Arginine (Arg)
Glycine (Gly)
Leucine (Leu)

While Applicants do not intend to be bound or limited by theory, the high level expression obtained by the present invention may be attributed to a wide range of factors. One possible explanation, for example, may be that the gene (DNA sequence) encoding protein derivatives have an impact on transcriptional efficiency, thereby creating increased copies of messenger RNA. Another explanation may be that the gene encoding protein derivatives have an impact on translational efficiency, thereby directly increasing polypeptide synthesis. Regardless of the theory or explanation, the protein derivatives of the present invention have higher levels of expression than their native protein counterparts. With the present invention, the ability to express desired proteins in high quantity is now especially applicable to small molecular weight proteins.

For purposes of the present invention, as disclosed and claimed herein, the following terms are as defined below.

Recombinant Protein - A protein expressed in a microorganism by virtue of the presence of a recombinant DNA vector that is autonomously replicating or integrated into the host cell genome.

Host Cell - A cell which has been or is capable of being transformed to express recombinant proteins.

Transformation - The introduction of DNA into a recipient host cell, including the viable protoplast thereof, that changes the genotype of the recipient cell.

## Description of the Figures

Figure 1 - Restriction site map of plasmid pKC283.
Figure 2 - Restriction site map of plasmid pKC283PX
Figure 3 - Restriction site map of plasmid pKC283-L.
Figure 4 - Restriction site map of plasmid pKC283-LB.

Figure 5 - Restriction site map of plasmid pKC283PRS.

Figure 6 - Restriction site map of plasmid pL32.

Figure 7 - Restriction site map of plasmid pNM789

Figure 8 - Schematic illustration of the construction protocol for plasmid p120.

Figure 9 - Restriction site map of plasmid pL47.

Figure 10 - Restriction site map of plasmid pPR12.

Figure 11 - Restriction site map of plasmid pPR12AR1.

Figure 12 - Restriction site map of plasmid pL110

Figure 13 - Schematic illustration of the construction protocol for plasmid pL110C.

Figure 14 - Restriction site map of plasmid pHS246.

The present invention discloses novel DNA sequences encoding protein derivatives that allow for increased expression of small molecular weight proteins. The novel derivatives have the formula Met-X-Trp-Protein and Met-X-X'-Protein wherein X and X' are selected from the group of amino acids consisting of methionine, phenylalanine, isoleucine, valine, serine, proline, threonine, alanine, glutamic acid, glycine, tyrosine, histidine, glutamine, asparagine, lysine, aspartic acid, cysteine, tryptophane, arginine, and leucine, and the protein is any biologically active sequence of amino acids ranging from about 1 to about 100 amino acids. The present invention further comprises related novel protein derivatives, linkers, expression vectors, and transformants thereof.

The present invention can be constructed and exemplified by transforming E. coli K12 RV308 with plasmid pHS246. Plasmid pHS246 codes for the expression of insulin-like growth factor II (IGFII). IGFII is a single polypeptide chain with three disulfide bonds and a molecular weight of 7471 Daltons. Insulin-like growth factor II is a member of the IGF family of growth promoting polypeptides with insulin-like activities. IGFII shows promise in the treatment of lowering blood glucose, promoting wound healing and inhibiting insulin secretion. Plasmid pHS246, which contains a tetracycline resistance gene of an expression cassette comprising the $\lambda P_L$ promoter and E. coli lipoprotein (lpp) ribosome binding site (rbs), was constructed by ligating fragments of plasmid pL110C and plasmid pHS190 to the synthetic linker sequence of the formula

$$CTAGAGGGTATTACATATGCGTTGGGCTTAT$$

$$TCCCATAATGTATACGCAACCCGAATAGC$$

wherein

A is deoxyriboadenyl,

G is deoxyriboguanyl,

C is deoxyribocytosyl, and

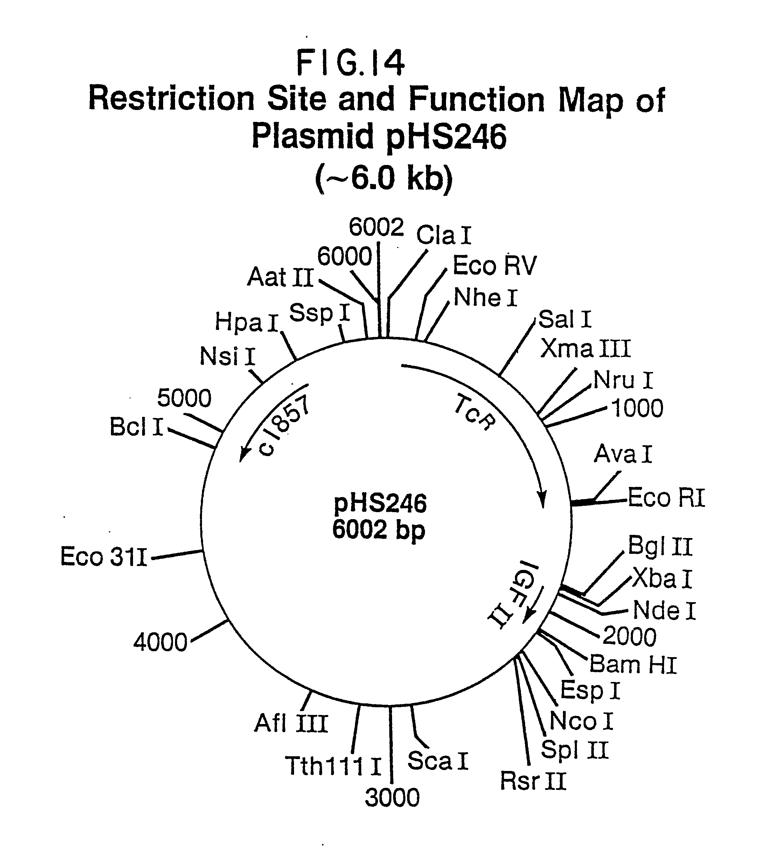

T is thymidyl The synthetic linker sequence can be constructed by use of the 380B DNA Synthesizer marketed by Applied Biosystems (850 Lincoln Drive, Foster City, CA 94404). or in accordance with the methods of phosphite triester synthesis (Caruthurs, M.H., Science 230, 281-285 (1985). A restriction site map of plasmid pHS246 is presented in Figure 14 of the accompanying drawings.

Additional IGFII derivatives containing different N-terminal sequences can be obtained by varying the first one or two codons following the initiation codon of the IGFII gene. Such derivatives are expressed at high levels in E. coli and are of the formula Met-X-Trp-IGFII and Met-X-X'-IGFII wherein X and X' are amino acids as described hereinabove. The particular sequence of nucleotides in the codons is important for eliciting high expression. Those skilled in the art will recognize that the degeneracy of the genetic code allows for an amino acid to be encoded by more than one codon. Consequently, codon choice as well as the particular sequence of amino acids encoded are both important factors in the generation of constructs for high level expression.

In the present invention, oligonucleotide linkers were synthesized which had the general formula methionine-x-tryptophane and methionine-x-x', wherein x and x' are any one of the 20 amino acids described above. In the synthesis of the oligonucleotides, the degeneracy of the genetic code was taken into account and the ability to select more than one codon for an amino acid was used to advantage. Restriction sites were also introduced into the oligonucleotides to allow for the easy insertion into plasmids containing the IGFII gene. Such oligonucleotides with properly designed restriction sites in conjunction with properly designed restriction sites in the IGFII gene-containing plasmid allow for conservation of the IGFII coding sequence as well as placing extra amino acids on the N-terminus.

Plasmid pL110C was used as the parental plasmid for constructing the IGFII expression plasmids of the present invention. Plasmid pL110C contains a $\lambda P_L$ promoter, an E. coli lpp ribosome binding site and a tetracycline resistance gene. The IGFII gene was isolated from plasmid pHS190 and given restriction sites to allow for correct placement into plasmid pL110C. After ligating together the IGFII gene, oligonucleotide linker, and plasmid pL110C, the expression vectors were complete. The IGFII expression vectors were then used to transform E. coli K12 RV308 cells (NRRL B-15624).

The small molecular weight polypeptides expressed as derivatives, are not limited solely to the IGFII protein. Other small molecular weight polypeptides, ranging from about 1 to about 100 amino acids, such as human proinsulin, human insulin A chain, human insulin B chain, human insulin-like growth factor I (IGFI), growth hormone releasing factor (GRF) and somatostatin may also be used for purposes of the present invention. Just as the IGFII gene, oligonucleotide and parental plasmid were ligated to allow generation of the many IGFII derivatives, coding sequences for other desired proteins may be employed in substantial accordance with the present teachings to generate other derivatives within the scope of the present invention.

The present invention is not limited solely to the use of plasmid pL110C. Any plasmid containing or designed to contain the appropriate promoter, not just $\lambda P_L$, but promoters like trp, lpp, and tac, may also be used. Such vectors include but are not limited to pBR322 and plasmid pCZR334. Just as the present invention is not limited to any particular plasmid or protein-encoding gene, the transformed host cell is not limited solely to E. coli K12 RV308. Any host cell that will allow expression of the desired gene may be used. Therefore, E. coli MM294, E. coli JM101, E. coli W3110, E. coli C600, E. coli WA704, Bacillus, Streptomyces, and yeast, for example, may also be used for purposes of the present invention. Several procedures well known in the art may be used to generate the desired extra amino acid sequence at the N-terminus of a given protein. Such procedures include producing such derivatives by classical solution phase, solid phase, or by recombinant DNA methodology.

IGFII and other protein derivatives have the potential to elicit undesirable immunological reactions when used in humans and other mammals. These reactions are believed to be caused by the extra amino acid sequences that are not present in the natural protein. In effect, the non-natural amino acid sequences are believed to be recognized in vivo as a foreign substance. Therefore, in order to eliminate the immunological response that the extra amino acid sequences elicit, it is desirable to cleave the extra amino acid sequences from the protein. Several cleavage methods, chemical or enzymatic, are well known in the art and may be employed to remove the extra amino acid sequences to generate non-immunogenic, native protein. Chemical agents useful for cleaving proteins are cyanogen bromide, 2-(2-nitrophenylsulfenyl)-3-bromo-3'-methylindolinium (BNPS-akatole), hydroxylamine, and the like. Cyanogen bromide cleaves proteins at the C-terminal of a methionine residue. Therefore, the selective cleavage site is a methionine residue itself. Hydroxylamine cleaves at the C-terminal of the moiety -Asn-Z- in which Z is Gly, Leu, or Ala. BNPS-akatole cleaves at the C-terminal of a tryptophan residue.

Examples of enzymatic agents useful for cleavage are trypsin, papain, pepsin, plasmin, thrombin, enterokinase, and the like. Each effects cleavage at a particular amino acid sequence which it recognizes. Enterokinase, for example, recognizes the amino acid sequence -(Asp)n-Lys- in which n is an integer from 2 to 4.

Edman degradation is another cleavage method which sequentially removes single N-terminal amino acids from the polypeptide. In the case of the present Met-x-Trp-IGFII derivatives, tryptophane oxidative cleavage can be used to cleave at the tryptophane site, thereby generating natural sequence IGFII. Example 33 details the procedure for obtaining native IGFII.

A particularly preferred protein for expression by use of the present invention is insulin-like growth factor II (IGFII). Insulin-like growth factor II is a member of the IGF family of growth promoting polypeptides with insulin-like activities. IGFII shows promise in the treatment of lowering blood glucose, promoting wound healing and inhibiting insulin secretion. A substantial increase in IGFII expression can be obtained by means of the present invention as compared to many methods for direct expression.

A flow diagram showing the construction of plasmid pL110C, the parental plasmid used for making constructs of the present invention, is presented in Table 1 below in outline form. Unless otherwise stated in the examples, the reaction conditions, buffers, and protocols for conventional recombinant DNA procedures such as restriction enzyme digestion, DNA fragment isolation and purification, ligation, and transformation are as disclosed in Maniatis, et al., 1982, Molecular Cloning (Cold Spring Harbor Laboratory), such disclosure being incorporated by reference herein.

## <u>Table 1</u>
### <u>Plasmid pL110 Construction Flow Diagram</u>

-Ex 1

pKC283     Ex 2     pKC283PX     Ex 6     pKC283PRS

-Ex 4

pKC283-L

-Ex 5

pKC283-LB

-Ex6

pL32

pNM789

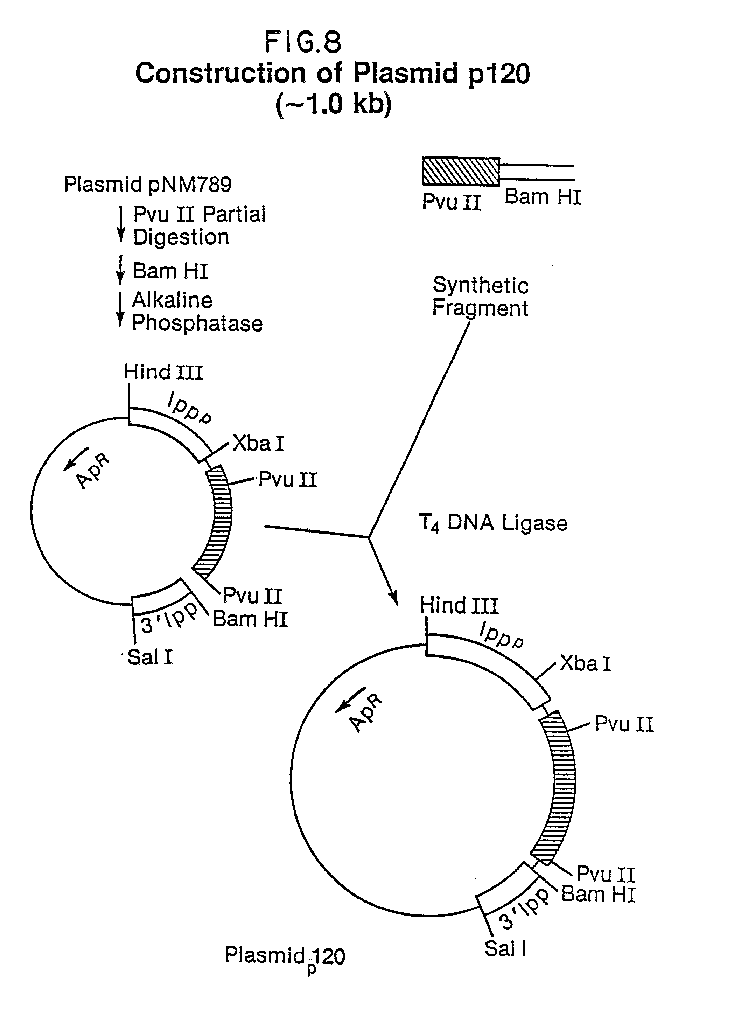

-Ex 7, Fig. 8

p120                             pPR12

-Ex 8

pPR12ΔR1

-Ex 8

-Ex 7            pPR12AR1

pL47

- Ex9

pL110

Ex 10-

pL110A
M13Tc110B
pL110C

The following examples further illustrate and detail the invention disclosed herein but are in no way intended to limit the scope of the invention. Both an explanation of and the actual procedures for constructing the invention are described where appropriate.

### Example 1

### <u>Isolation of Plasmid pKC283</u>

Lyophils of E. coli K12 BE1201/pKC283 are obtained from the Northern Regional Research Laboratory, Peoria, Illinois 61604, under the accession number NRRL B-15830. The lyophils are decanted into tubes containing 10 ml LB medium (10 g Bacto-tryptone, 5 g Bacto-yeast extract, and 10 g NaCl per liter; pH is adjusted to 7.5) and incubated two hours at 32°C, at which time the cultures are made 50 µg/ml in ampicillin and then incubated at 32°C overnight. The E. coli K12 BE1201/pKC283 cells were cultured at 32°C, because the cells comprise a temperature-sensitive cl repressor gene integrated into the cellular DNA. When cells that comprise a wild-type lambda pL repressor gene or do not comprise a lambda pL promoter are utilized in this plasmid isolation procedure, as described in subsequent examples herein, the temperature of incubation is 37°C.

A small portion of the overnight culture is placed on LB-agar (LB medium with 15 g/l Bacto-agar) plates containing 50 µg/ml ampicillin in a manner so as to obtain a single colony isolate of E. coli K12 BE1201/pKC283. The single colony obtained was inoculated into 10 ml of LB medium containing 50 µg/ml ampicillin and incubated overnight at 32°C with vigorous shaking. The 10 ml overnight culture was inoculated into 500 ml LB medium containing 50 µg/ml ampicillin and incubated at 32°C with vigorous shaking until the culture reached stationary phase.

The following procedure is adapted from Maniatis et al., 1982, Molecular Cloning (Cold Spring Harbor Laboratory).

The cells were harvested by centrifugation at 4000Xg for 10 minutes at 4°C, and the supernatant was discarded. The cell pellet was washed in 100 ml of ice-cold STE buffer (0.1 M NaCl; 10 mM Tris-HCl, pH 7.8; and 1 mM EDTA). After washing, the cell pellet was resuspended in 10 ml of Solution 1 (50 mM glucose; 25 mM Tris-HCl, pH 8.0; and 10 mM EDTA) containing 5 mg/ml lysozyme and left at room temperature for 10 minutes. Twenty ml of Solution 2 (0.2 N NaOH and 1% SDS) were then added to the lysozyme-treated cells, and the solution was gently mixed by inversion. The mixture was incubated on ice for 10 minutes.

Fifteen ml of ice-cold 5 M potassium acetate, pH 4.8, were added to the lysed-cell mixture and the solution mixed by inversion. The solution was incubated on ice for 10 minutes. The 5 M potassium acetate solution was prepared by adding 11.5 ml of glacial acetic acid to 28.5 ml of water and 60 ml of 5 M potassium acetate; the resulting solution is 3 M with respect to potassium and 5 M with respect to acetate.

The lysed cell mixture was centrifuged in a Beckman SW27 (or its equivalent) at 20,000 rpm for 20 minutes at 4°C. The cell DNA and debris formed a pellet on the bottom of the tube. About 36 ml of supernatant were recovered, and 0.6 volumes of isopropanol were added, mixed, and the resulting solution left at room temperature for 15 minutes. The plasmid DNA was collected by centrifugation at 12,000Xg for 30 minutes at room temperature. The supernatant was discarded, and the DNA pellet was washed with 70% ethanol at room temperature. The ethanol wash was decanted, and the pellet was dried in a vacuum desiccator. The pellet was then resuspended in 8 ml of TE buffer (10 mM Tris-HCl, pH 8.0, and 1 mM EDTA).

Eight grams of CsCl were added to the DNA solution. About 0.8 ml of a 10 mg/ml solution of ethidium bromide in water were added for each 10 ml of CsCl-DNA solution. The final density of the solution was about 1.55 g/ml, and the ethidium bromide concentraton was about 600 µg/ml. The solution was transferred to a Beckman Type 50 centrifuge tube, filled to the top with paraffin oil, sealed, and centrifuged at 45,000 rpm for 24 hours at 20°C. After centrifugation, two bands of DNA were visible in ordinary light. After removing the cap from the tube, the lower DNA band was removed by using a syringe with a #21 hypodermic needle inserted through the side of the centrifuge tube.

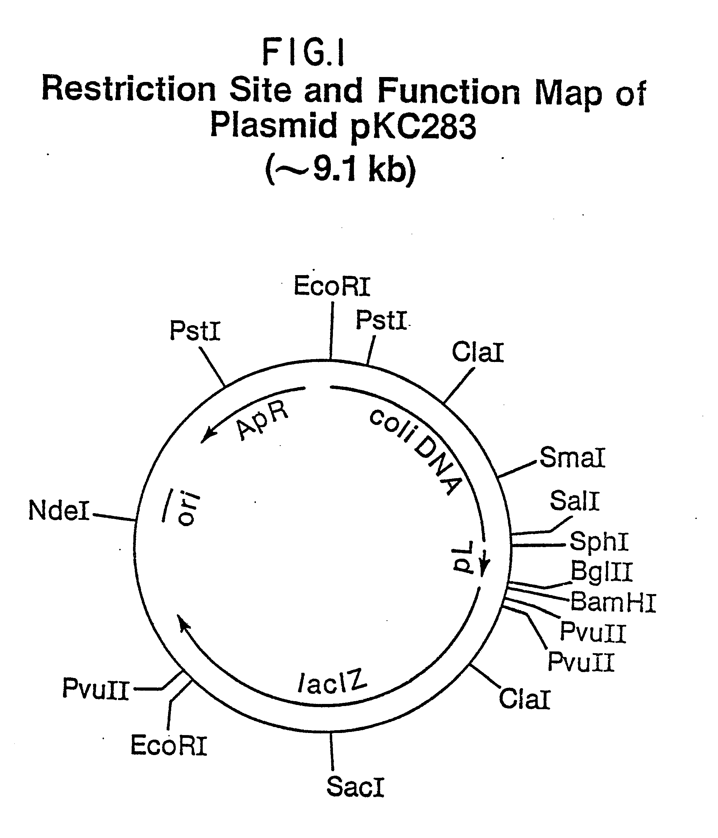

The ethidium bromide was removed by several extractions with water-saturated 1-butanol. The CsCl was removed by dialysis against TE buffer. After extractions with buffered phenol and then chloroform, the DNA was precipitated, washed with 70% ethanol, and dried. About 1 mg of plasmid pKC283 was obtained and stored at 4°C in TE buffer at a concentration of about 1 µg/µl. A restriction site and function map of plasmid pKC283 is presented in Figure 1 of the accompanying drawings.

Example 2

Construction of Plasmid pKC283PX

About 10 μl of the plasmid pKC283 DNA prepared in Example 1 were mixed with 20 μl 10 X medium-salt restriction buffer (500 mM NaCl; 100 mM Tris-HCl, pH 7.5; 100 mM MgCl₂; and 10 mM DTT), 20 μl 1 mg/ml BSA, 5 μl restriction enzyme PvuII (~50 Units, as defined by Bethesda Research Laboratories (BRL), from which all restriction enzymes used herein were obtained), and 145 μl of water, and the resulting reaction was incubated at 37°C for 2 hours. Restriction enzyme reactions described herein were routinely terminated by phenol and then chloroform extractions, which were followed by precipitation of the DNA, an ethanol wash, and resuspension of the DNA in TE buffer. After terminating the PvuII digestion as described above, the PvuII-digested plasmid pKC283 DNA was precipitated and then resuspended in 5 μl of TE buffer.

About 600 picomoles (pM) of XhoI linkers (5′-CCTCGAGG-3′) were kinased in a mixture containing 10 μl 5 X Kinase Buffer (300 mM Tris-HCl, pH 7.8; 50 mM MgCl₂; and 25 mM DTT), 5 μl 5 mM ATP, 24 μl H₂O, 0.5 μl of T4 polynucleotide kinase (about 2.5 units as defined by P-L Biochemicals), 5 μl 1 mg/ml BSA, and 5 μl of 10 mM spermidine by incubating the mixture at 37°C for 30 minutes.

About 12.5 μl of the kinased XhoI linkers were added to the 5 μl of PvuII-digested plasmid pKC283 DNA, and then 2.5 μl of 10 X ligase buffer (300 mM Tris-HCl, pH 7.6; 100 mM MgCl₂; and 50 mM DTT), 2.5 μl of 1 mg/ml BSA, 7 μl of 5 mM ATP, 2.5 μl (about 2.5 units as defined by P-L Biochemicals) of T4 DNA ligase, 2.5 μl of 10 mM spermidine, and 3 μl of water were added to the DNA. The resulting ligation reaction was incubated at 4°C overnight. After the ligation reaction, the reaction mixture was adjusted to have the composition of high-salt buffer (0.1 M NaCl; 0.05 M Tris-HCl, pH 7.5; 10.0 mM MgCl₂; and 1 mM DTT). About 10 μl (100 units) of restriction enzyme XhoI were added to the mixture, and the resulting reaction was incubated at 37°C for 2 hours.

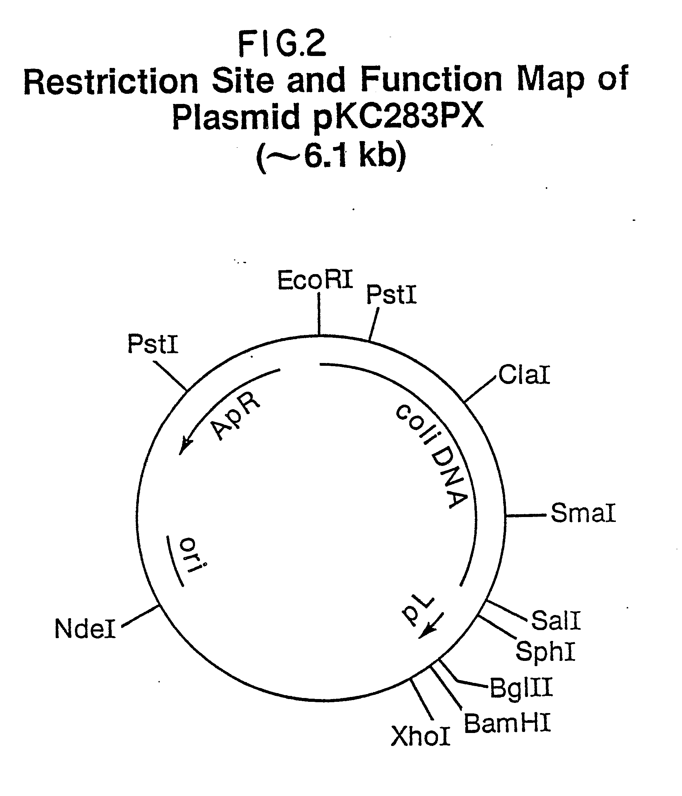

The reaction was terminated, and the XhoI-digested DNA was precipitated, resuspended, and ligated as described above, except that no XhoI linkers were added to the ligation mixture. The ligated DNA constituted the desired plasmid pKC283PX. A restriction site and function map of plasmid pKC283PX is presented in Figure 2 of the accompanying drawings.

## Example 3

## Construction of E. coli K12 MO(λ⁺)/pKC283PX

E. coli K12 MO(λ⁺) can be obtained from the Northern Regional Research Laboratories in lyophylized form under the accession number NRRL B-15993. E. coli K12 MO(λ⁺) comprises the wild-type lambda pL cl repressor gene, so that transcription from the hybrid pL-lpp promoter of the present invention does not occur in E. coli K12 MO(λ⁺) cells. The lyophils are reconstituted, single colonies of MO(λ⁺) are isolated, and a 10 ml overnight culture of the MO(λ⁺) cells is prepared in substantial accordance with the procedure of Example 1, except that the temperature of incubation is 37°C and no ampicillin is used in the growth media.

Fifty μl of the overnight culture were used to inoculate 5 ml of LB media which also contained 10 mM MgSO₄ and 10 mM MgCl₂. The culture was incubated at 37°C overnight with vigorous shaking. The following morning, the culture was diluted to 200 ml with LB media containing 10 mM MgSO₄ and 10 mM MgCl₂. The diluted culture was incubated at 37°C with vigorous shaking until the absorbance at 550 nm (A₅₅₀) was about 0.5, which indicated a cell density of about 1 x 10⁸ cells/ml. The culture was cooled for ten minutes in an ice-water bath, and the cells were then collected by centrifugation at 4000Xg for 10 minutes at 4°C. The cell pellet was resuspended in 100 ml of cold 10 mM MgSO₄ and then immediately repelleted by centrifugation. The cell pellet was resuspended in 100 ml of 30 mM CaCl₂ and incubated on ice for 20 minutes.

The cells were again collected by centrifugation and resuspended in 10 ml of 30 mM CaCl₂. A one-half ml aliquot of the cells was added to the ligated DNA prepared in Example 2; the DNA had been made 30 mM in CaCl₂. The cell-DNA mixture was incubated on ice for one hour, heat-shocked at 42°C for 90 seconds, and then chilled on ice for about two minutes. The cell-DNA mixture was diluted into 10 ml of LB media in 125 ml flasks and incubated at 37°C for one hour. One hundred μl aliquots were plated on LB-agar plates containing ampicillin and incubated at 37°C until colonies appeared.

The colonies were individually cultured, and the plasmid DNA of the individual colonies was examined by restriction enzyme analysis and gel electrophoresis. Plasmid DNA isolation was performed on a smaller scale in accordance with the procedure of Example 1, but the CsCl gradient step was omitted until the desired E. coli K12 MO($\lambda^+$)/pKC283PX transformants were identified. A restriction site and function map of plasmid pKC283PX is presented in Figure 2 of the accompanying drawings.

## Example 4

## Construction of E. coli K12 MO($\lambda^+$)/pKC283-L

Ten $\mu$g of plasmid pKC283PX DNA prepared in accordance with the procedure of Example 1 were dissolved in 20 $\mu$l of 10X high-salt buffer (1M NaCl, 5M Tris-HCl (pH 7.5), .1M MgCl$_2$ and .01M DTT), 20 $\mu$l 1 mg/ml BSA, 5 $\mu$l (~50 units) restriction enzyme BglII, 5$\mu$l (~50 units) restriction enzyme XhoI, and 150 $\mu$l of water, and the resulting reaction was incubated at 37°C for two hours. The reaction was stopped, and after precipitating the BglII-XhoI digested DNA, the DNA was resuspended in 5 $\mu$l of TE buffer.

A DNA linker with single-stranded DNA ends characteristic of BglII and XhoI restriction enzyme cleavage was synthesized and kinased. The linker was kinased in substantial accordance with the procedure of Example 2. The DNA linker had the following structure:

$$5'-GATCTATTAACTCAATCTAGAC-3'$$
$$| | | | | | | | | | | | | | | | | | | |$$
$$3'-ATAATTGAGTTAGATCTGAGCT-5'$$

The linker depicted above was synthesized from single-stranded deoxyoligonucleotides by procedures well known in the art. The single-stranded deoxyoligonucleotides can be synthesized with commercially available instruments, such as the 380A DNA Synthesizer marketed by Applied Biosystems (850 Lincoln Centre Drive, Foster City, CA 94404), which utilizes phosphoramidite chemistry. Other procedures for synthesizing DNA are also known in the art. The conventional modified phosphotriester method of synthesizing single stranded DNA is described in Itakura et al., 1977, Science 198:1056 and in Crea et al., 1978, Proc. Nat. Acad. Sci. USA 75:5765. In addition, an especially preferred method of synthesizing DNA is disclosed in Hsiung et al., 1983, Nucleic Acid Research 11:3227 and Narang et al., 1980, Methods in Enzymology 68:90.

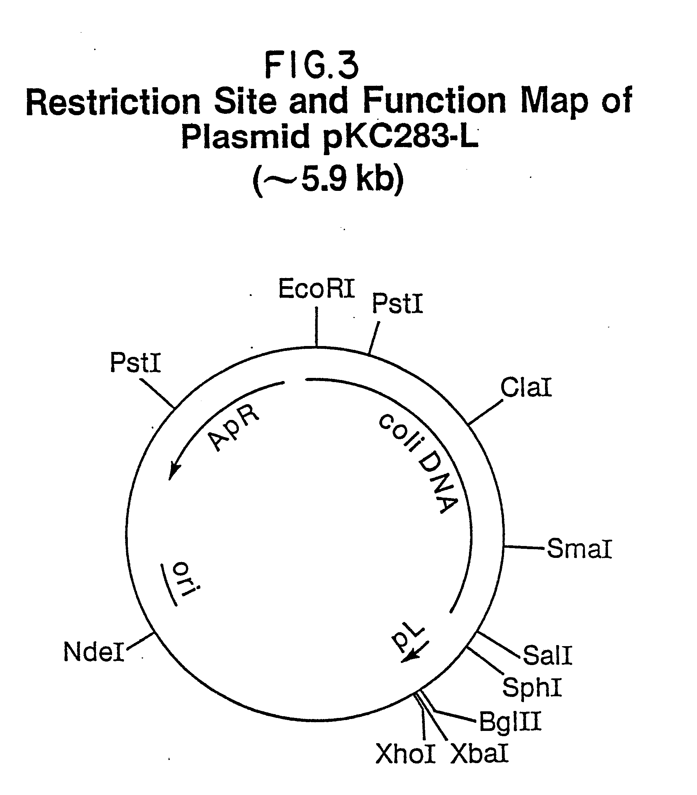

The linker and BglII-XhoI-digested plasmid pKC283PX were ligated in substantial accordance with the procedure of Example 2. The ligated DNA constituted the desired plasmid pKC283-L. A restriction site and function map of plasmid pKC283-L is presented in Figure 3 of the accompanying drawings. The plasmid pKC283-L DNA was used to transform E. coli K12 MO($\lambda^+$) and the resulting E. coli K12 MO($\lambda^+$)/pKC283-L transformants were identified in substantial accordance with the procedure of Example 3.

## Example 5

## Construction of E. coli K12 MO($\lambda^+$)/pKC283-LB

About 10 $\mu$g of plasmid pKC283-L DNA, prepared in substantial accordance with the procedures of Example 1, were dissolved in 20 $\mu$l 10X high-salt buffer, 20 $\mu$l 1 mg/ml BSA, 5 $\mu$l (~50 units) restriction enzyme XhoI, and 155 $\mu$l of H$_2$O, and the resulting reaction was incubated at 37°C for two hours. The XhoI-digested plasmid pKC283-L DNA was then precipitated from the reaction mixture by the addition of three volumes of 95% ethanol and one-tenth volume of 3 M sodium acetate, incubation in a dry ice-ethanol bath for five minutes, and centrifugation. The resulting DNA pellet was washed with 70% ethanol, dried, and resuspended in 2 $\mu$l 10X nick-translation buffer (0.5 M Tris-HCl, pH 7.2; 0.1 M MgSO$_4$; and 1 mM DTT), 1 $\mu$l of a solution 2 mM in each of the deoxynucleotide triphosphates, 15 $\mu$l of H$_2$O, 1 $\mu$l (~6 units as defined by P-L Biochemicals) of Klenow, which is the large fragment of E. coli DNA polymerase I, and 1 $\mu$l of 1 mg/ml BSA. The resulting reaction was incubated at 25°C for 30 minutes; the reaction was stopped by incubating the solution at 70° C for five minutes.

BamHI linkers (5´-CGGGATCCCG-3´) were kinased and ligated to the XhoI-digested, Klenow-treated plasmid pKC283-L DNA in substantial accordance with the procedure of Example 2. After the ligation reaction, the DNA was digested with about 100 units of BamHI for about 2 hours at 37° C in high-salt buffer. After the BamHI digestion, the DNA was prepared for ligation in substantial accordance with the procedure of Example 2.

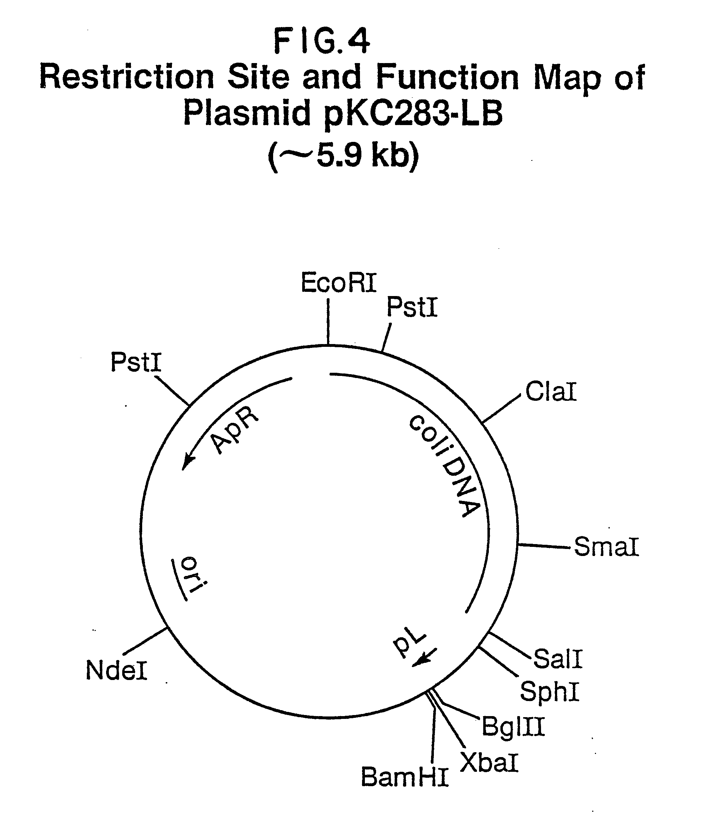

The ~5.9 kb BamHI restriction fragment was circularized by ligation and transformed into E. coli K12 MO(λ⁺) in substantial accordance with the procedures of Examples 2 and 3. The E. coli K12 MO(λ⁺)-/pKC283-LB transformants were identified, and then plasmid pKC283-LB DNA was prepared in substantial accordance with the procedure of Example 1. A restriction site and function map of plasmid pKC283-LB is presented in Figure 4 of the accompanying drawings.


## Example 6


## Construction of E. coli K12 MO(λ⁺)/pL32


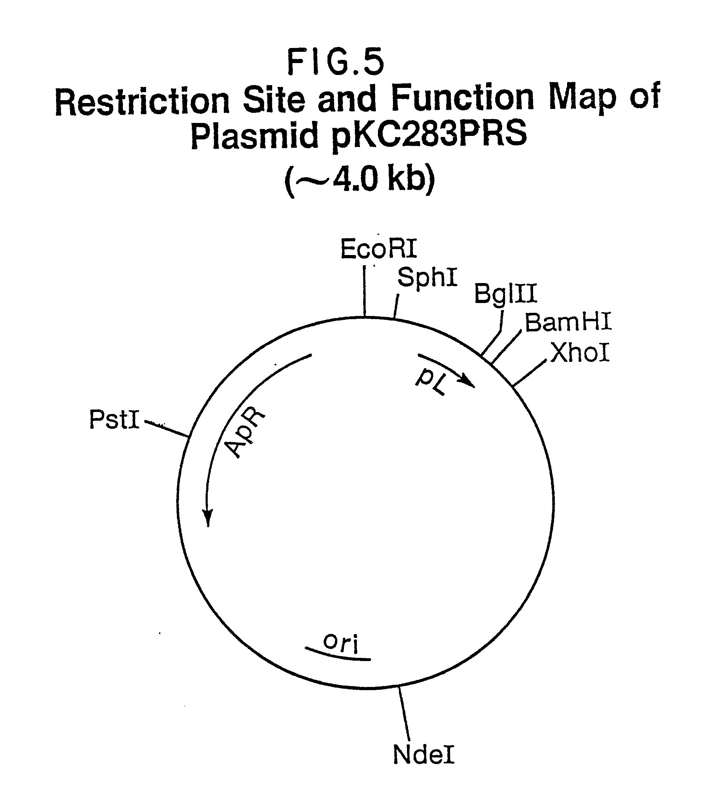

About 10 µg of plasmid pKC283PX were digested with restriction enzyme SalI in high-salt buffer, treated with Klenow, and ligated to EcoRI linkers (5´-GAGGAATTCCTC-3´) in substantial accordance with the procedure of Example 5, with the exception of the starting plasmid, restriction enzymes, and linkers used. After digestion with restriction enzyme EcoRI, which results in the excision of ~2.1 kb of DNA, the ~4.0 kb EcoRI restriction fragment was circularized by ligation to yield plasmid pKC283PRS. The ligated DNA was used to transform E. coli K12 MO(λ⁺) in substantial accordance with the procedure of Example 3. After the E. coli K12 MO(λ⁺)/pKC283PRS transformants were identified, plasmid pKC283PRS DNA was prepared in substantial accordance with the procedure of Example 1. A restriction site and function map of plasmid pKC283PRS is presented in Figure 5 of the accompanying drawings.

About 10 µg of plasmid pKC283PRS were digested in 200 µl of high-salt buffer with about 50 units each of restriction enzymes PstI and SphI. After incubating the reaction at 37° C for about 2 hours, the reaction mixture was electrophoresed on a 0.6% low-gelling-temperature agarose (FMC Corporation, Marine Colloids Division, Rockland, Maine 04841) gel for 2-3 hours at ~130 V and ~75 mA in Tris-Acetate buffer.

The gel was stained in a dilute solution of ethidium bromide, and the band of DNA constituting the ~0.85 kb PstI-SphI restriction fragment, which was visualized with long-wave UV light, was cut from the gel in a small segment. The volume of the segment was determined by weight and density of the segment, and an equal volume of 10 mM Tris-HCl, pH 7.6, was added to the tube containing the segment. The segment was then melted by incubation at 72° C. About 1 µg of the ~0.85 kb PstI-SphI restriction fragment of plasmid pKC283PRS was obtained in a volume of about 100 µl. In an analogous manner, plasmid pKC283-LB was digested with restriction enzymes PstI and SphI, and the resulting ~3.0 kb restriction fragment was isolated by agarose gel electrophoresis and prepared for ligation.

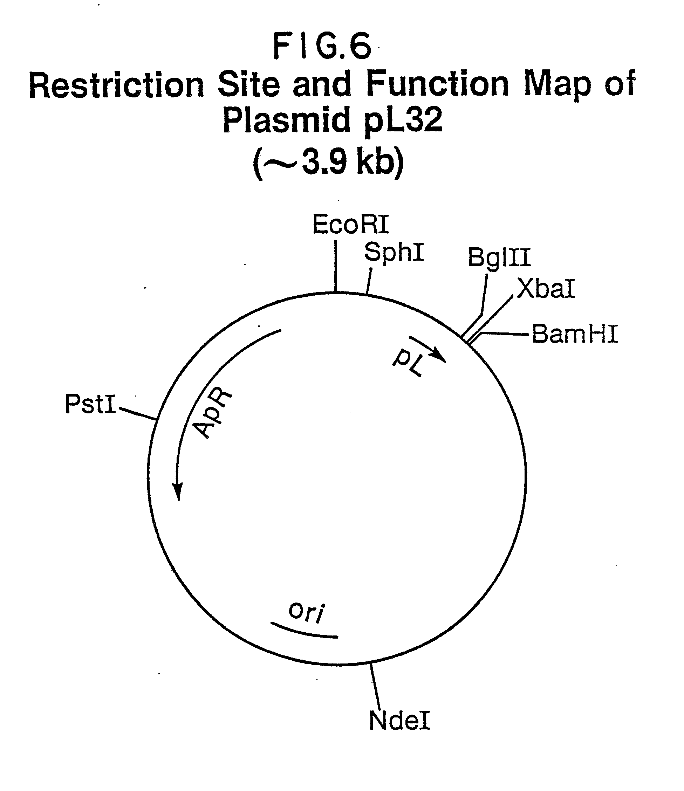

The ~0.85 kb PstI-SphI restriction fragment of plasmid pKC283PRS was ligated to the ~3.0 kb PstI-SphI restriction fragment of plasmid pKC283-LB in substantial accordance with the procedure of Example 2. The ligated DNA constituted the desired plasmid pL32. A restriction site and function map of plasmid pL32 is presented in Figure 6 of the accompanying drawings. Plasmid pL32 was transformed into E. coli K12 MO-(λ⁺) cells in substantial accordance with the procedure of Example 3. Plasmid pL32 DNA was prepared from the E. coli K12 MO(λ⁺)/pL32 transformants in substantial accordance with the procedure of Example 1. Analysis of the plasmid pL32 DNA demonstrated that more than one EcoRI linker attached to the Klenow-treated, SalI ends of plasmid pKC283PX. The presence of more than one EcoRI linker does not affect the utility of plasmid pL32 or derivatives of plasmid pL32 and can be detected by the presence of an XhoI restriction site, which is generated whenever two of the EcoRI linkers are ligated together. Alternatively, plasmid pL32 may be constructed by carrying out the SalI-EcoRI excision and ligation of the first paragraph of this Example upon plasmid pKC283-LB.


## Example 7

## Construction of E. coli K12 MO($\lambda^+$)/pL47

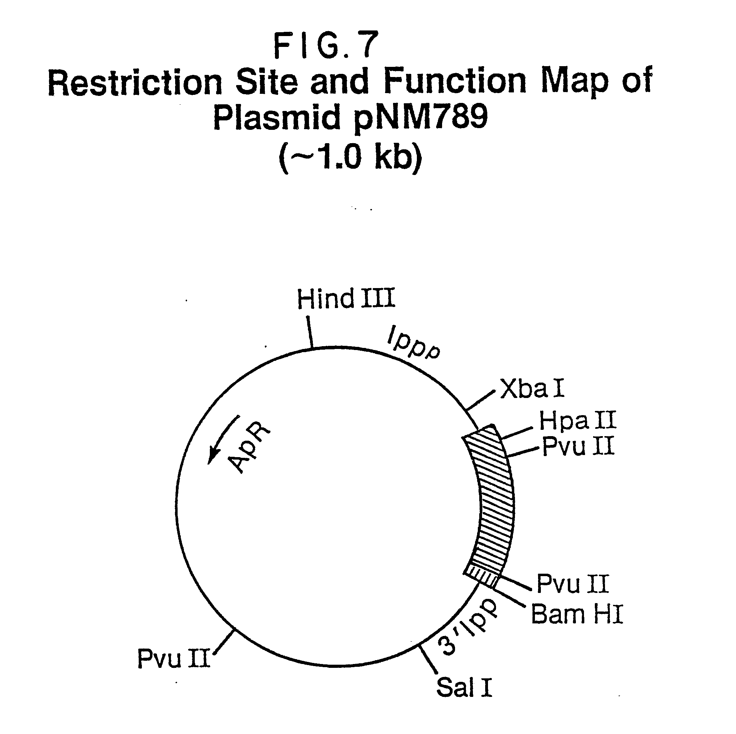

E. coli K12 RV308/pNM789 can be obtained from the Northern Regional Research Laboratories in lyophilized form under the accession number NRRL B-18216. A restriction site and function map of pNM789 is presented in Figure 7 of the accompanying drawings. Plasmid DNA is extracted from the culture in substantial accordance with the teaching of Example 1, except that the temperature of incubation is 37° C. Ten micrograms of pNM789 are suspended in 200 $\mu$l PvuII buffer (50 mM Tris-HCl (pH 7.5), 60 mM NaCl and 6mM MgCl$_2$). One unit of PvuII is added and the reaction mix is incubated for 5 minutes at 37° C. The enzyme is inactivated by heating 10 minutes at 65° C. 30 $\mu$l of 10X BamHI buffer (200 mM Tris-HCl (pH 8.0), 1M NaCl and 70 mM MgCl$_2$), 70 $\mu$l H$_2$O and 10 units of BamHI are next added and the reaction is incubated for 1 hour at 37° C. This is followed by the addition of 5 units of alkaline phosphatase and incubation for 1 hour at 65° C. The DNA fragments are separated on a 1 percent agarose gel, and a DNA fragment (Figure 8) the size of a single cut fragment is purified.

A synthetic DNA linker with a blunt PvuII end and a BamHI end is synthesized in substantial accordance with the teaching of Example 4. This linker has the following structure:

$$5\text{'}-CTGTGCCTTCTAG-3\text{'}$$
$$| | | | | | | | | | | | |$$
$$3\text{'}-GACACGGAAGATCCTAG-5\text{'}$$

The linker is kinased and ligated into the BamHI-PvuII digested plasmid pNM789 in substantial accordance with the teaching of Example 2. This ligation mixture is used to transform E. coli K12 RV308 cells and plasmid isolation is performed upon these transformants in substantial accordance with the teaching of Example 3. Several plasmids are selected which contain the appropriate size PvuII fragment (494bp) and XbaI-BamHI fragment (628bp). The sequence of at least two of these is determined by sequencing from the BamHI site toward the unique SmaI site and one clone is selected with the desired sequence. This intermediate plasmid is designated plasmid p120. A schematic outline of this procedure and a restriction site and function map of plasmid p120 is presented in Figure 8 of the accompanying drawings.

To isolate the BGH-encoding DNA, about 10 $\mu$g of plasmid p120 were digested in 200 $\mu$l of high-salt buffer containing about 50 units each of restriction enzymes XbaI and BamHI. The digestion products were separated by agarose gel electrophoresis, and the ~0.6 kb XbaI-BamHI restriction fragment which encodes BGH was isolated and prepared for ligation in substantial accordance with the procedure of Example 6.

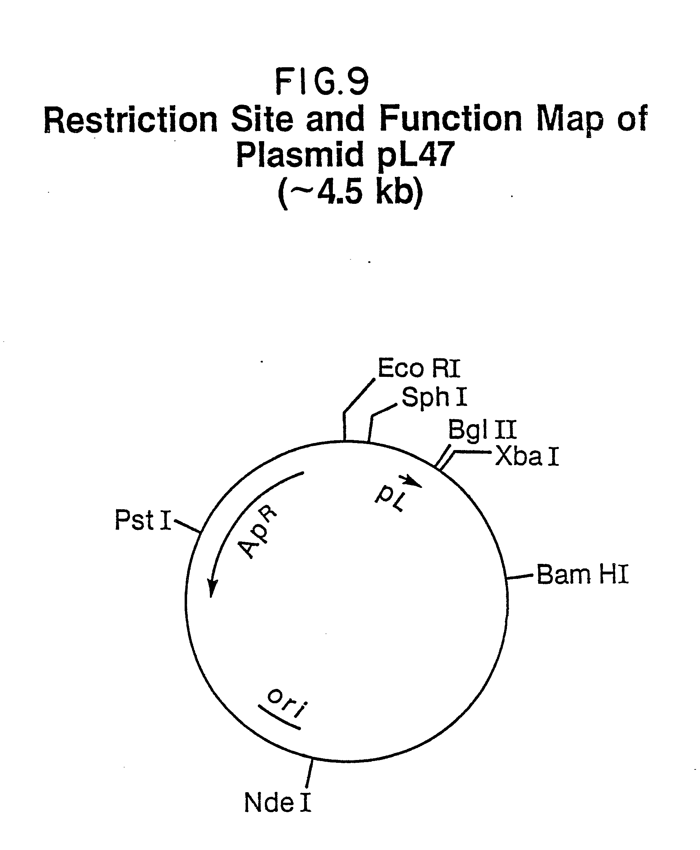

Plasmid pL32 was also digested with restriction enzymes XbaI and BamHI, and the ~3.9 kb restriction fragment was isolated and prepared for ligation. The ~3.9 kb XbaI-BamHI restriction fragment of plasmid pL32 was ligated to the ~0.6 kb XbaI-BamHI restriction fragment of plasmid p120 in substantial accordance with the procedure of Example 2 to yield plasmid pL47. A restriction site and function map of plasmid pL47 is presented in Figure 9 of the accompanying drawings. Plasmid pL47 was transformed into E. coli K12 MO($\lambda^+$) in substantial accordance with the procedure of Example 3, and the E. coli K12 MO($\lambda^+$)/pL47 transformants were identified. Plasmid pL47 DNA was prepared from the transformants in substantial accordance with the procedures of Example 1.

## Example 8

## Construction of E. coli K12 RV308/pPR12AR1

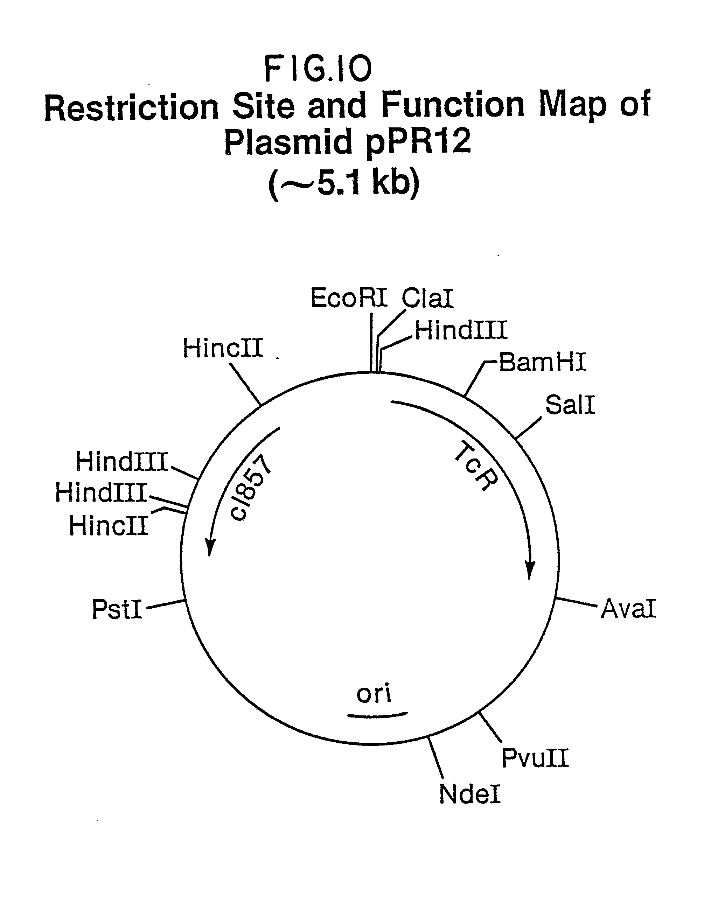

Plasmid pPR12 comprises the temperature-sensitive pL repressor gene cl857 and the plasmid pBR322 tetracycline resistance-conferring gene. Plasmid pPR12 is disclosed and claimed in U.S. Patent No. 4,436,815, issued 13 March 1984. A restriction site and function map of plasmid pPR12 is presented in Figure 10 of the accompanying drawings.

About 10 $\mu$g of plasmid pPR12 were digested with about 50 units of restriction enzyme EcoRI in 200 $\mu$l of high-salt buffer at 37° C for two hours. The EcoRI-digested plasmid pPR12 DNA was precipitated and treated with Klenow in substantial accordance with the procedure of Example 5. After the Klenow reaction, the EcoRI-digested, Klenow-treated plasmid pPR12 DNA was recircularized by ligation in substantial accordance with the procedure of Example 2. The ligated DNA, which constituted the desired plasmid pPR12ΔR1, was used to transform E. coli K12 RV308 in substantial accordance with the procedure of Example 3, except that selection was based on tetracycline (5 μg/ml) resistance, not ampicillin resistance. E. coli K12 RV308 is available from the NRRL under the accession number NRRL B-15624. After the E. coli K12 RV308/pPR12ΔR1 transformants were identified, plasmid pPR12ΔR1 DNA was prepared from the transformants in substantial accordance with the procedure of Example 1.

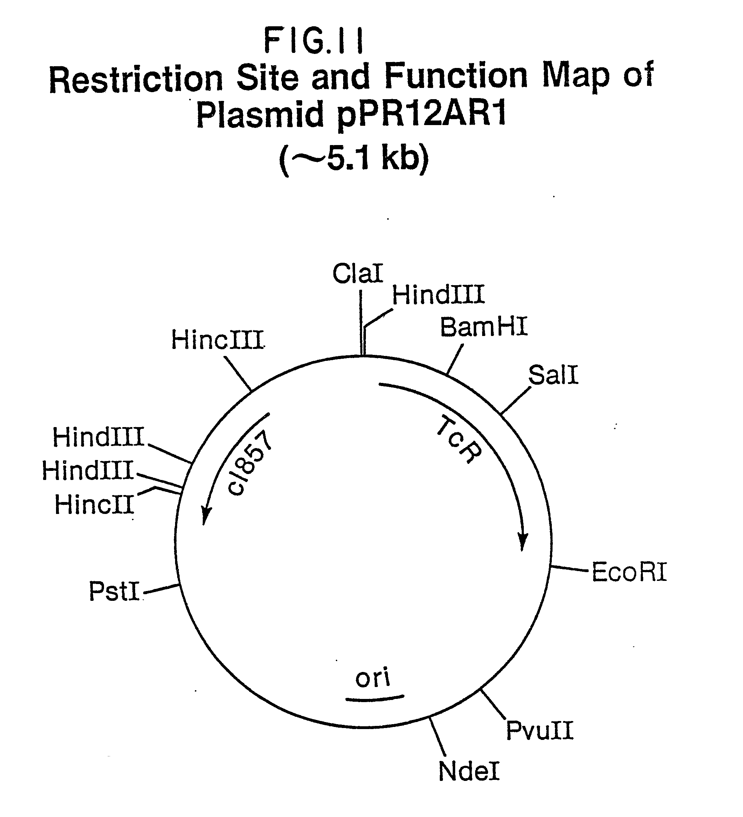

About 10 μg of plasmid pPR12ΔR1 were digested with about 50 units of restriction enzyme AvaI in 200 μl of medium-salt buffer at 37°C for 2 hours. The AvaI-digested plasmid pPR12ΔR1 DNA was precipitated and treated with Klenow in substantial accordance with the procedure of Example 5. After the Klenow reaction, the AvaI-digested, Klenow-treated plasmid pPR12ΔR1 DNA was ligated to EcoRI linkers (5′-GAGGAATTCCTC-3′) in substantial accordance with the procedure of Example 2. After the linker ligation, the DNA was precipitated and then resuspended in about 200 μl of high-salt buffer containing about 50 units of restriction enzyme EcoRI. The resulting reaction was incubated at 37°C for about 2 hours. After the EcoRI digestion, the reaction mixture was loaded onto an agarose gel, and the ~5.1 kb EcoRI restriction fragment was purified in substantial accordance with the procedure of Example 6. The ~5.1 kb EcoR1 restriction fragment was recircularized by ligation in substantial accordance with the procedure of Example 2. The ligated DNA constituted the desired plasmid pPR12AR1. The plasmid pPR12AR1 DNA was transformed into E. coli K12 RV308 in substantial accordance with the procedure of Example 3, except that selection was based on tetracycline resistance, not ampicillin resistance. After identifying the E. coli K12 RV308/pPR12AR1 transformants, plasmid pPR12AR1 DNA was prepared in substantial accordance with the procedure of Example 1. A restriction site and function map of plasmid pPR12AR1 is presented in Figure 11 of the accompanying drawings.

## Example 9

## Construction of E. coli K12 RV308/pL110

About 10 μg of plasmid pPR12AR1 DNA were suspended in about 200 μl of high-salt buffer containing about 50 units each of restriction enzymes PstI and EcoRI, and the digestion reaction was incubated at 37°C for about 2 hours. The reaction mixture was then loaded onto an agarose gel, and the ~2.9 kb PstI-EcoR1 restriction fragment of plasmid pPR12AR1 was isolated and prepared for ligation in substantial accordance with the procedure of Example 6.

About 10 ug of plasmid pL47 were digested with restriction enzymes PstI and BamHI in 200 μl of high-salt buffer at 37°C for two hours. The PstI-BamHI-digested DNA was loaded onto an agarose gel, and the ~2.7 kb PstI-BamHI restriction fragment that comprised the origin of replication and a portion of the ampicillin resistance-conferring gene was isolated and prepared for ligation in substantial accordance with the procedure of Example 6. In a separate reaction, about 10 μg of plasmid pL47 DNA were digested with restriction enzymes EcoRI and BamHI in 200 μl of high-salt buffer at 37°C for two hours, and the ~1.03 kb EcoRI-BamHI restriction fragment that comprised the novel transcriptional and translational activating sequence and the BGH-encoding DNA was isolated and prepared for ligation in substantial accordance with the procedure of Example 6. The ~2 μg of the ~1.03 kb EcoRI-BamHI restriction fragment obtained were used in the construction of plasmid pL110.

The ~2.7 kb PstI-BamHI and ~1.03 kb EcoRI-BamHI restriction fragments of plasmid pL47 were ligated to the ~2.9 kb PstI-EcoRI restriction fragment of plasmid pPR12AR1 to construct plasmid pL110, and the ligated DNA was used to transform E. coli K12 RV308 in substantial accordance with the procedure of Examples 2 and 3, except that tetracycline resistance, not ampicillin resistance, was used as the basis for selecting transformants.

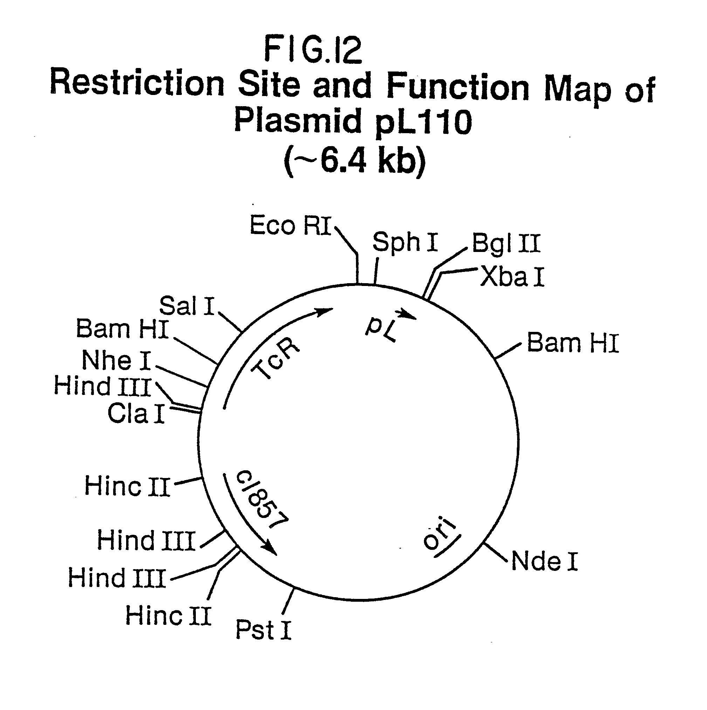

Two PstI restriction enzyme recognition sites are present in the BGH coding region that are not depicted in the restriction site and function maps presented in the accompanying drawings. A restriction site and function map of plasmid pL110 is presented in Figure 12 of the accompanying drawings.

## Example 10

11

Construction of E. coli K12 RV308/pL110C

A. Construction of E. coli K12 RV308/PL110A

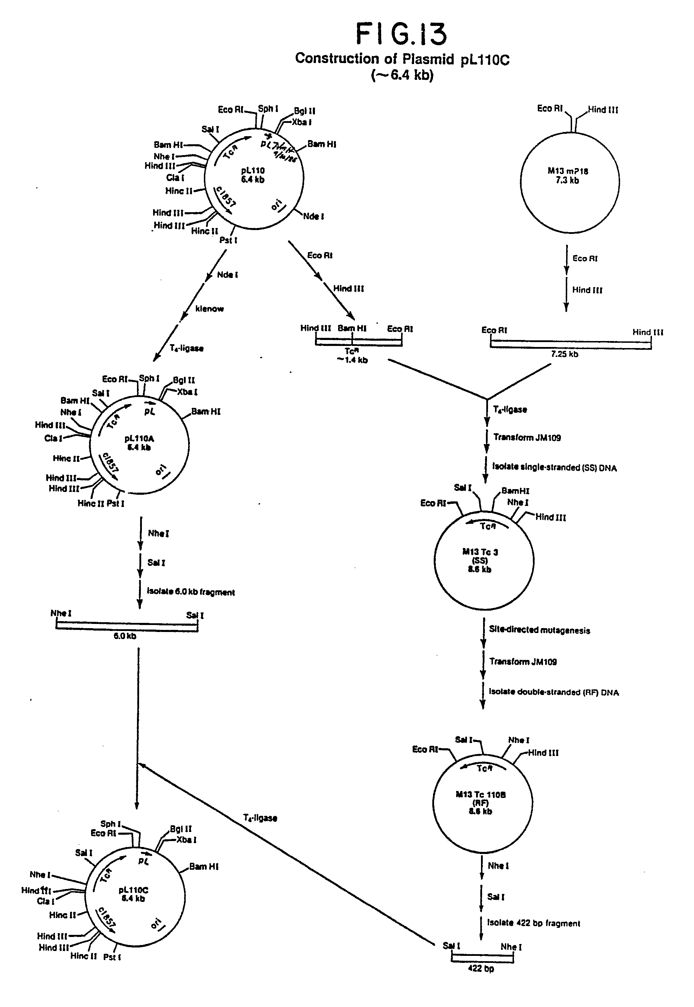

About 1 μg of plasmid pL110 DNA was digested with restriction enzyme NdeI in 20 μl total volume containing 2 μl of 10X high-salt buffer (1.0 M NaCl; 0.50 M Tris-HCl, pH = 7.5; 0.10 M MgCl₂; and 10 mM dithiothreitol) and 3 units of NdeI enzyme for 1 hour at 37° C. The reaction mixture was extracted with phenol/chloroform mixture (1:1, v) and the DNA precipitated with ethanol. The NdeI-digested plasmid pL110 DNA was dissolved in 50 μl of 1X Klenow buffer (40 mM KPO₄, pH = 7.5; 6.6 mM MgCl₂; 1.0 mM 2-mercaptoethanol; 33 μM dATP; 33 μM dCTP; 33 μM dGTP; and 33 μM TTP). Two μl (~10 units, New England Biolabs) of the large fragment of E. coli DNA polymerase I, known as Klenow, were added to and mixed with the DNA, and the resulting reaction was incubated at 16° C for 1 hour. The reaction was terminated by phenol/CHCl₃ extraction and the DNA conventionally purified. The NdeI-digested, Klenow-treated DNA was then ligated with T4 DNA ligase at 4° C for 16 hours. The resulting DNA was used to conventionally transform E. coli K12 strain RV308 (NRRL B-15624). Transformants were selected on TY-agar plates containing 5 μg/ml tetracycline and plasmids isolated from resistant colonies by the rapid alkaline extraction procedure described by Birnboim and Doly. (Nucleic Acids Res. 7, 1513-23 (1979)) A plasmid (pL110A in Figure 13) lacking an NdeI site was selected.

B. Construction of Phage M13Tc110B by Site-Specific Mutagenesis

The protocol for eliminating the BamHI site in the tetracycline resistance-conferring gene by site-specific mutagenesis is shown on the right hand side of Figure 13 of the accompanying drawings.

B(i) Construction of Phage M13Tc3

Plasmid pL110 served as the source of the tetracycline resistance-conferring gene. About 50 μg of plasmid pL110 in 50 μl of TE buffer were added to 25 μl of 10X HindIII buffer and 170 μl of H2O. About 5 μl (~50 units) of restriction enzyme HindIII were added to the solution of plasmid pL110 DNA, and the resulting reaction was incubated at 37° C for 2 hours. About 13 μl of 2 M Tris.HCl, pH = 7.4, and 5 μl (~50 units) of restriction enzyme EcoRI were added to the HindIII-digested plasmid pL110 DNA, and the reaction was incubated for 2 more hours at 37° C. The reaction was stopped by extracting the reaction mixture with TE-saturated phenol; the phenol was removed by chloroform extractions. The EcoRI-HindIII-digested plasmid pL110 DNA was then collected by precipitation and centrifugation, loaded into a 1% agarose gel, and the 1.4 kb EcoRI-HindIII restriction fragment was purified and isolated.

About 5 μg of phage M13mp18 (New England Biolabs) were dissolved in 50 μl of TE buffer and then digested with HindIII and EcoRI as described above. The HindIII-EcoRI-cut phage M13mp18 DNA was purified as described for pL110 except that an ~7.25 kb restriction fragment was purified and isolated.

About 100 nanograms of the ~1.4 kb HindIII-EcoRI fragment of plasmid pL110 were mixed with about 400 nanograms of the ~7.3 kb HindIII-EcoRI fragment of phage M13mp18, 2 μl of 10X ligase buffer (0.5M Tris (pH 7.4), 0.1M MgCl₂, 0.1M dithiothreitol, 10mM spermidine, 10mM ATP, and 1 mg/ml BSA), 1 μl (~1 unit) of T4 DNA ligase, and 14 μl of H₂O. The ligation reaction was incubated at 15° C for 1.5 hours; the ligated DNA constituted the desired phage M13Tc3 DNA.

One ml of an overnight culture of E. coli K12 JM109 (E. coli K12 JM101, available from New England Biolabs, can be used instead of E. coli K12 JM109) was used to inoculate 50 ml of L broth, and the resulting culture was incubated at 37° C with aeration until the O.D.₆₆₀ was between 0.3 and 0.4. The cells were resuspended in 25 ml of 10 mM NaCl, incubated on ice for 10 minutes, and collected by centrifugation. The cells were resuspended in 1.25 ml of 75 mM CaCl₂; a 200 μl aliquot of the cells was removed, added to 10 μl of the ligated DNA prepared above, and incubated on ice for about 40 minutes. The cell-DNA mixture was then incubated at 42° C for 2 minutes, and varying aliquots (1, 10, and 100 μl) were removed and added to 3 ml of top agar (L broth with 0.5% agar kept molten at 45° C) that also

contained 50 μl of 2% X-Gal, 50 μl of 100 mM IPTG, and 200 μl of E. coli K12 JM109 in logarithmic growth phase. The cell-top agar mixture was then plated on L-agar plates containing 40 μg/ml X-Gal (5-bromo-4-chloro-3-indolyl-β-D-thiogalactoside) and 0.1 mM IPTG (isopropyl-β-D-thiogalactoside), and the plates were incubated at 37°C overnight.

The following morning, several clear, as opposed to blue, plaques were individually picked to inoculate 2 ml of L broth, and the resulting cultures were incubated at 37°C with aeration for 2 hours. Then, the cultures were centrifuged, and 200 μl of the resulting supernatant were added to 10 ml cultures (O.D.$_{550}$ = 0.5) of E. coli K12 JM109 growing at 37°C with aeration. These cultures were incubated for another 30 minutes at 37°C; then, the cells were pelleted by centrifugation and used to prepare the replicative-form of the recombinant phage they contained. Double-stranded, replicative form phage DNA was isolated from the cells using a scaled-down version of the procedure described in Example 1. Transformants containing phage M13Tc3 DNA were identified by restriction enzyme analysis of their phage DNA.

B(ii) Preparation of Single-Stranded Phage M13Tc3 DNA

One and one-half ml of an overnight culture of E. coli K12 JM109/M13Tc3 were centrifuged, and 100 μl of the phage M13Tc3-containing supernatant were used to inoculate a 25 ml culture of E. coli JM109 at an O.D.$_{660}$ of about 0.4-0.5. The culture was incubated for 6 hours at 37°C with aeration, at which time the culture was centrifuged and the resulting supernatant, about 20 ml, transferred to a new tube. About 2 ml of a solution containing 20% polyethylene glycol (PEG 6000) and 14.6% NaCl were added to the supernatant, which was then incubated on ice for 20 minutes.

The supernatant was centrifuged for 25 minutes at 7000 rpm, and the resulting pellet, which contained single-stranded phage M13Tc3 DNA, was resuspended in 500 μl of TE buffer. The DNA solution was extracted twice with TE-saturated phenol and twice with chloroform. The single-stranded DNA was then precipitated using NaOAc and ethanol and centrifuged. The resulting pellet was washed with 70% ethanol, dried, and then dissolved in 60 μl of H$_2$O.

B(iii) Mutagenesis

The single-stranded DNA fragment used as the mutagenesis primer was synthesized on an automated DNA synthesizer. The mutagenesis primer has the sequence, 5'-CCCGTCCTGTGGATACTCTACGCCGGA-3', and is homologous to the region surrounding the BamHI site (5'-GGATCC-3') in the tetracycline resistance-conferring gene from plasmid pBR322, except that the second A residue from the 5' end of the primer (or third A residue from the 3' end) is a C in plasmid pBR322. This change does not alter the amino acid composition of the tetracycline resistance-conferring protein but eliminates the BamHI site.

About 10 picomoles of the mutagenic primer and the M13 universal primer (Bethesda Research Laboratories (BRL), P.O. Box 6009, Gaithersburg, MD 20760) were individually treated with 1 unit (BRL) of T4 polynucleotide kinase in 20 μl of 1X kinase buffer (60 mM Tris-HCl, pH = 7.8; 15 mM 2-mercaptoethanol; 10 mM MgCl$_2$; and 0.41 μM ATP) for 30 minutes at 37°C. The kinase-treated DNAs were used in the mutagenesis procedure described below.

The annealing reaction was carried out mixing together 300 nanograms (1.2 μl) of single-stranded phage M13Tc3, 1 picomole (2 μl) of the universal primer, 1 picomole (2 μl) of the mutagenic primer, 2 μl of 10X annealing buffer (100 mM Tris-HCl, pH = 7.5; 1 mM EDTA; and 500 mM NaCl), and 12.8 μl of H$_2$O. The reaction was incubated at 80°C for 2 minutes, at 50°C for 5 minutes, and then allowed to cool to room temperature.

The extension reaction was carried out by adding 5 μl of 10X extension buffer (500 mM Tris-HCl, pH = 8; 1 mM EDTA; and 120 mM MgCl$_2$); 5 μl of 2 mM dATP; 1 μl of a solution 6 mM in each of dGTP, TTP, and dCTP; 1 μl (~2 units, Pharmacia P-L Biochemicals, 800 Centennial Avenue, Piscataway, NJ 08854) of Klenow enzyme; 1 μl (1 unit, Bethesda Research Laboratories, Gaithersburg, MD 20877) of T4 DNA ligase; and 17 μl of H$_2$O to the mixture of annealed DNA. The extension reaction was incubated at room temperature for 1 hour, then at 37°C for 2.5 hours, and then overnight at 4°C.

The reaction was stopped by two extractions with TE-saturated phenol, which were followed by two extractions with CHCl$_3$. The DNA was precipitated with ethanol and NaOAc. The DNA was collected by centrifugation and resuspended in 50 μl of H$_2$O, and 6 μl of 10X S1 buffer were then added to the solution of DNA.

The solution of DNA was split equally into three tubes. About 200 units (Miles Laboratories) of S1 nuclease were added to two of the tubes. One S1 reaction was incubated at room temperature for 5 minutes, the other for 10 minutes. The reactions were stopped by extracting the reaction mixture twice with TE-saturated phenol. The phenol extractions were followed by two extractions with chloroform; then, the DNA was precipitated from the reaction mixture with NaOAc and ethanol. The untreated sample of DNA served as a negative control. The S1-treated samples were kept separate from each other throughout the remainder of the procedure but gave similar results.

The DNA pellets were resuspended in 20 μl of H2O, and 10 μl of the resulting solution were used to transform E. coli K12 JM109 (E. coli K12 JM101 could also be used) in accordance with the procedure used during the construction of phage M13Tc3, except that no IPTG or X-Gal was added to the plates.

Double-stranded replicative form DNA from about 48 plaques was isolated as described above and screened for the presence of a BamHI restriction site. Isolates without a BamHI site were further screened by preparing single-stranded DNA as described above. The single-stranded DNA was sequenced using the dideoxy sequencing method (Andrew J.H. Smith, 1980, Methods in Enzymology 65: 560-580). The desired isolate was designated M13Tc110B (Figure 13).

## C. Construction of Plasmid pL110C

About 50 μg of the replicative form of phage M13Tc110B DNA were digested in 250 μl of 1X NheI buffer (50 mM NaCl; 6 mM Tris•HCl, pH = 7.5; 6 mM MgCl₂; and 6 mM β-mercaptoethanol) containing ~50 units of NheI restriction enzyme at 37°C for 2 hours. Five μl of 5 M NaCl were then added to the NheI-digested phage M13Tc110B DNA, followed by 5 μl (~50 units) of SalI restriction enzyme. Digestion was continued for 2 hours at 37°C. The desired ~422 bp NheI-SalI fragment containing the mutated region of the tetracycline resistance-conferring gene was then isolated from an acrylamide gel, according to the teaching of Example 11.

Plasmid pL110A DNA was digested with NheI and SalI under identical conditions, except that plasmid pL110A was substituted for phage M13Tc110B. The ~6.0 kb NheI-SalI restriction fragment of plasmid pL110A was purified from agarose.

The desired plasmid pL110C was constructed by ligating together 100 nanograms each of the NheI-SalI fragments of pL110A (~6.0 kb) and M13Tc110B (~422 bp) using conventional procedures. A restriction site and function map of plasmid pL110C is presented in Figure 13 of the accompanying drawings. The desired plasmid pL110C confers tetracycline resistance to 10 μg/ml tetracycline in E. coli but lacks a BamHI site in the tetracycline resistance-conferring gene.

## Example 11

## Preparation of Oligonucleotide Linker

The linker sequence

CTAGAGGGTATTACATATGCGTTGGGCTTAT
TCCCATAATGTATACGCAACCCGAATAGC

was prepared by first synthesizing single stranded oligonucleotides on a Applied Biosystems Inc. 380B DNA Synthesizer and then kinasing each synthetic single-stranded oligonucleotide with T4-DNA kinase in a standard kinasing buffer (50mM Tris-HCl (pH 7.5), 10mM MgCl₂, 1mM EDTA, 10mM DTT dithiothreitol) followed by reannealing equal-molar synthetic oligonucleotides. Reannealing was performed by heating two complementary synthetic oligonucleotides to 80°C for 5 minutes in a polypropylene centrifuge tube (15 ml) or eppendorf tube (1.5 ml) and then allowing the sample to slowly cool to 4°C.

## Example 12

Construction of Expression Plasmid pHS246 and E. coli K12 RV308/pHS246

A. Preparation of pL110C Xbal/BamHI Fragment (5770 bp)

About 50 μl of (100 μg) plasmid pL110C DNA was mixed with about 10 μl of 10X M Salt buffer (50mM NaCl, 10mM Tris-HCl (pH 7.9) 10mM MgCl₂), 30 μl of H₂O, 5 μl of Xbal (20 μ/μl) and 5 μl of BamHI (25 μ/μl) restriction enzyme. The mixture was incubated at 37°C for 2 hours. The 5770 bp restriction fragment was purified by .7% Low melting agarose gel electrophoresis procedure (SEA Plaque Agarose, FMC Bioproducts) and precipitated with ethanol. The pellet was resuspended in 40 μl of TE (10mM Tris, 1mM EDTA, pH 8.0) and stored at 0°C for future use.

B. EcoRI/BamHI Digestion of Plasmid pHS190

Plasmid pHS190 can be obtained from the deposited strain (NRRL B-18410) (E. coli K12 RV308/pHS190) and isolated in substantial accordance with the teaching of Example 2A. About 43 μl of plasmid pHS190 was digested by adding 10 μl of 10X EcoRI buffer (100mM Tris-HCl, pH 7.5, 50mM NaCl, 10mM MgCl₂), 40 μl of H₂O, 3.5 μl of EcoRI restriction enzyme (20 μ/μl) and 3.5 μl of BamHI restriction enzyme (25 units/μl). The mixture was incubated at 37°C for 2 hours. The restriction fragments were purified on a 4% NuSieve gel and the 660 bp fragment isolated and precipitated with ethanol. This fragment contains the IGFII gene.

C. Taql Digestion of the 660 bp Fragment of Plasmid pHS190

The ≈ 660 bp IGFII EcoRI/BamHI fragment, obtained in part B, was digested with Taql restriction enzyme. Thus, about 40 μl of the EcoRI/BamHI fragment, 5 μl of the 10X Taql buffer (50mM Tris-HCl (pH 8.0), 10mM MgCl₂, 50mM NaCl), and 4 μl of Taql (10 μ/μl) restriction enzyme were mixed and incubated at 65°C for 2 hours. The restriction fragments were purified on a 4% NuSieve gel and the 201 bp fragment isolated and resuspended in TE buffer.

D. Ligation

Approximately 10 pmoles of the oligonucleotide linker, obtained in Example 1, .02 μg of the Taql/BamHI digested pHS190 fragment, obtained in part C, and .4 μg of the Xbal/BamHI digested pL110C fragment, obtained in part A, were mixed in a 20 μl standard ligation buffer (50mM Tris-HCl, pH 7.5, 10mM MgCl₂, 10mM DTT, 1mM EDTA, .5mM ATP) and 1 unit of T4-DNA ligase (BRL) was added allowed to react at 12°C for 16 hours. The resultant plasmid is designated plasmid pHS246. About 7.5 μl of the ligation mixture was used to transform 200 μl of frozen competent E. coli K12 RV308 cells (NRRL 15624) following standard procedures as set out in Maniatis, et al., 1982, Molecular Cloning (Cold Spring Harbor Laboratory). The transformants were grown at 32°C on TY tetracycline plates containing 5 μg/ml tetracycline. Colonies were picked from the plate and cultures grown at 32°C for 12-16 hours. Cultures were inoculated 1:50 with 12-16 hour cultures and grown while shaking at 32°C until an OD ≈ .3 was reached. Once the correct OD was reached, the temperature was shifted to 42°C and the cells incubated for 2-3 hours. Raising the temperature to 42°C induces the λp_L promoter, thus resulting in high level expression of the desired Met-Arg-Trp-IGFII derivative.

Additional plasmids coding for IGFII derivatives of the formula Met-X-trp-IGFII can be constructed in substantial accordance with the teaching of Examples 11 and 12 except that the linker sequence

CTAGAGGGTATTACATATGCGTTGGGCTTAT

TCCCATAATGTATACGCAACCCGAATAGC

is replaced with the linker sequence

CTAGAGGGTATTACATATATGATG    TGGGCTTAT
-R-
TCCCATAATGTATATAC    ACCCGAATAGC

wherein R is defined in the following Table 1. The resultant plasmids and related IGFII protein derivatives are also presented for completeness.

## Table 1

|  | Linker Sequence | Plasmid | Protein Derivative |
|---|---|---|---|
| Ex. 3 | R is CGA GCT | pHS274 | met-arg-trp-IGFII |
| Ex. 4 | R is TTA AAT | pHS273 | met-leu-trp-IGFII |
| Ex. 5 | R is ATG TAC | pHS247 | met-met-trp-IGFII |
| Ex. 6 | R is CAA GTT | pHS249 | met-gln-trp-IGFII |
| Ex. 7 | R is CAT GTA | pHS278 | met-his-trp-IGFII |
| Ex. 8 | R is TGG ACC | pHS272 | met-trp-trp-IGFII |

| Ex. 9 | R is GTA CAT | pHS276 | met-val-trp-IGFII |
|---|---|---|---|
| Ex. 10 | R is TTG AAC | pHS270 | met-leu-trp-IGFII |
| Ex. 11 | R is TTT AAA | pHS281 | met-phe-trp-IGFII |
| Ex. 12 | R is AGC TCG | pHS275 | met-ser-trp-IGFII |
| Ex. 13 | R is TCC AGG | pHS277 | met-ser-trp-IGFII |
| Ex. 14 | R is CAC GTG | pHS283 | met-his-trp-IGFII |
| Ex. 15 | R is CGG GCC | pHS282 | met-arg-trp-IGFII |
| Ex. 16 | R is CCG GGC | pHS271 | met-pro-trp-IGFII |
| Ex. 17 | R is GCA CGT | pHS280 | met-ala-trp-IGFII |

Table 2 lists additional examples of transformants that can be constructed in substantial accordance with the teaching of Example 12.

## Table 2

### Example 18

Construction of E. coli $R^2/pR^3$ wherein $R^2$ is RV308, MM294, C600, JM101 or W3110 and $R^3$ is independently plasmids pHS274, pHS273, pHS247, pHS249, pHS278, pHS272, pHS276, pHS270, pHS281, pHS275, pHS297, pHS283, pHS282, pHS271, or pHS280. Transformation is carried out in substantial accordance with the teaching of Example 12.

Additional plasmids coding for IGFII derivatives of the general formula met-X-X'-IGFII can be constructed in substantial accordance with the teaching of Examples 11 and 12 except that the linker sequence

CTAGAGGGTATTACATATGCGTTGGGCTTAT

TCCCATAATGTATACGCAACCCGAATAGC

17

is replaced with the linker sequence

$$CTAGAGGGTATTACATATG \quad\quad GCTTAT$$
$$-Z-Z'-$$
$$TCCCATAATGTATAC \quad\quad CGAATAGC$$

wherein Z and Z' are as defined in the following Table 3. The resultant plasmids and related IGFII protein derivatives are also presented for completeness.

## Table 3

| Linker Sequence | | | Plasmid | Protein Derivative |
|---|---|---|---|---|
| Ex. 19 | Z is TCC AGG | Z' is CAC GTG | pHS250 | met-ser-his-IGFII |
| Ex. 20 | Z is AAG TTC | Z' is GCT CGA | pHS251 | met-lys-ala-IGFII |
| Ex. 21 | Z is ATC TAG | Z' is GAG CTC | pHS290 | met-ile-glu-IGFII |
| Ex. 22 | Z is ATG TAC | Z' is CAA GTT | pHS291 | met-met-gln-IGFII |

```
Ex. 23   Z   is  ATA            pHS292         met-ile-trp-IGFII
                 TAT

         Z'  is  TGG
                 ACC


Ex. 24   Z   is  AAT            pHS293         met-asn-leu-IGFII
                 TTA

         Z'  is  CTG
                 GAC


Ex. 25   Z   is  CAT            pHS253         met-his-ala-IGFII
                 GTA

         Z'  is  GAC
                 CTG


Ex. 26   Z   is  GCT            pHS294         met-ala-lys-IGFII
                 CGA

         Z'  is  AAA
                 TTT


Ex. 27   Z   is  TAT            pHS295         met-tyr-asn-IGFII
                 ATA

         Z'  is  AAC
                 TTG


Ex. 28   Z   is  AGG            pHS296         met-arg-asn-IGFII
                 TCC

         Z'  is  AAC
                 TTG
```

```
Ex. 29   Z   is  TTA          pHS297          met-leu-val-IGFII
                 AAT
             Z'  is  GTT
                     CAA


Ex. 30   Z   is  CCT          pHS298          met-pro-thr-IGFII
                 GGA
             Z'  is  ACA
                     TGT


Ex. 31   Z   is  ACT          pHS299          met-thr-arg-IGFII
                 TGA
             Z'  is  CGG
                     GCC
```

Table 4 lists additional examples of transformants that can be constructed in substantial accordance with the teaching of Example 12.

Table 4

Example 32

Construction of E. coli $R^4$/p$R^5$

wherein $R^4$ is RV308, MM294, C600, WA704, JM101 or W3110 and $R^5$ is independently plasmids pHS250, pHS251, pHS290, pHS291, pHS292, pHS293, pHS253, pHS294, pHS295, pHS296, pHS297, pHS298, or pHS299.

Example 33

Cleavage of met-x-trp-IGFII to Yield Natural Sequence IGFII

Cleavage Reaction

Lyophilized granules containing Met-X-Trp-IGF-II were used as the starting material for tryptophan oxidative cleavage reactions to liberate IGFII. 100 mg of dried granules were dissolved in 9.6 ml of 90% formic acid in a round bottom flask. After stirring at room temperature for two hours, 100 $\mu$l each of DMSO and concentrated HCl were added and allowed to stir for 30 minutes. Another 100 $\mu$l each of DMSO and concentrated HCl were added and allowed to stir for another 30 minutes. The reaction mixture was then rotoevaporated to dryness. The dried material was suspended in 50 ml of Milli Q water and lyophilized. The dried cleaved material was then taken up in 7.5M urea, 0.5M Tris base pH 8.2 containing 0.1M $Na_2SO_3$ and 0.01M $Na_2SO_3$ to convert the IGFII present to the S-sulfonate form for analysis by reverse-phase (RP) HPLC and Mono S cation exchange chromatography. An alternate tryptophan oxidative cleavage reaction carried out with Met-X-Trp-IGF-II containing granules was taken from the literature (Schechter, Y., Patchornik A., and Burstein, Y. Biochemistry, (1976) 15, 5071-5075) with the following modifications. Urea was included in the reaction mixture to increase the accessibility of the Trp residue. Briefly, 500 mg of dried granules were dissolved in 50 ml of 50% acetic acid/3.75M urea with stirring at room temperature for three hours. Three 2.0 ml aliquots of a 100mM solution of NCS (N-chlorosuccinimide) in DMF (N-dimethylforamide) were added 20 minutes apart to give a total reaction time of one hour. Methionine (9 ml of a 200mM solution) was added to stop the reaction. The samples were rotoevaporated and analyzed as described above.

Analysis of Cleavage Products

The IGFII (SSO$_3$)$_6$ generated by the tryptophan oxidative cleavage reactions described above was analyzed by RP HPLC and Mono S cation exchange chromatography. The RP HPLC system used Zorbax C8 150 A double end-capped 25cm X 4mm column (Dupont, Wilmington, DE) at 45° C. The A solvent was 50mM Tris-H$_3$PO$_4$ pH 7.8 and the B solvent was 50mM Tris-H$_3$PO$_4$ pH 7.8, 50% CH$_3$CN. A gradient was run from 25% B to 65% B over 30 minutes at 1 ml/min. The Mono S analytical column was run on a LCC500 FPLC from Pharmacia.. The A buffer was 20mM citric acid, 7M urea, pH 3.5 and the B buffer was 20mM citric acid, 7M urea, pH 3.5, 1.0M NaCl. A gradient was run from 0% to 40% B over 30 minutes at 1 ml/min.

Amino acid analysis of S-carboxymethyl derivatives of IGFII prepared as described by Means, G. E. and Feeney, R. E., in Chemical Modification of Proteins, pp. 218-219 (Holden-Day, Inc. Publisher, 1971) were carried out on a Beckmann 6300 High Performance Amino Acid Analyzer, to measure the amount of cysteic acid formation.

**Claims**

1. A DNA sequence encoding methionine-x-x′-protein, wherein x and x′ are selected from the group consisting of phenylalanine, leucine, isoleucine, methionine, valine, serine, proline, threonine, alanine, tyrosine, histidine, glutamine, aparagine, lysine, apartic acid, glutaminc acid, cysteine, tryptophane, arginine and glycine.

2. A DNA sequence of Claim 1 wherein the protein is IGFII, somatostatin, insulin A chain, insulin B chain, IGFI or GRF.

3. A DNA sequence of Claim 2 wherein the protein is IGFII.

4. The DNA sequence of Claim 1 wherein x is arginine and x′ is tryptophane.

5. A DNA sequence of Claim 1 wherein x is serine and x′ is histidine.

6. A DNA sequence of Claim 1 wherein x is lysine and x′ is alanine.

7. A DNA sequence of Claim 1 wherein x is isoleucine and x′ is glutamic acid.

8. A DNA sequence of Claim 1 wherein x is methionine and x′ is glutamine.

9. A DNA sequence of Claim 1 wherein x is isoleucine and x′ is tryptophane.

10. A DNA sequence of Claim 1 wherein x is asparagine and x′ is leucine.

11. A DNA sequence of Claim 1 wherein x is arginine and x′ is serine.

12. A DNA sequence of Claim 1 wherein x is histidine and x′ is alanine.

13. A DNA sequence of Claim 1 wherein x is alanine and x′ is lysine.

14. A DNA sequence of Claim 1 wherein x is tyrosine and x′ is asparagine.

15. A DNA sequence of Claim 1 wherein x is arginine and x′ is asparagine.

16. A DNA sequence of Claim 1 wherein x is leucine and x′ is valine.

17. A DNA sequence of Claim 1 wherein x is proline and x′ is threonine.

18. A DNA sequence of Claim 1 wherein x is threonine and x′ is arginine.

19. A method for producing a recombinant protein, the method comprising transforming a host cell with a recombinant DNA expression vector which comprises

a) a DNA sequence encoding methionine-x-x′ protein, wherein x and x′ are selected from the group consisting of phenylalanine, leucine, isoleucine, methionine, valine, serine, proline, threonine, alanine, tyrosine, histidine, glutamine, asparagine, lysine, aspartic acid, glutamic acid, cysteine, tryptophane, arginine and glycine, and

b) culturing the transformed cell under conditions suitable for gene expression.

20. The method of Claim 19 in which the protein is IGFII, somatostatin, insulin A chain, insulin B chain, IGFI or GRF.

21. A recombinant DNA expression vector which comprises a DNA sequence encoding methionine-x-x′-protein, wherein x and x′ are selected from the group consisting of phenylalanine, leucine, isoleucine, methionine, valine, serine, proline, threonine, alanine, tyrosine, histidine, glutamine, asparagine, lysine, aspartic acid, glutamic acid, cysteine, tryptophane, argine and glycine.

22. The vector of Claim 21 which is a plasmid.

23. The vector of Claim 22 which is plasmid pHS246.

24. A host cell comprising the vector of Claim 21, 22 or 23.

25. The host cell of Claim 24 which is E. coli.

26. The host cell of Claim 25 which is E. coli K12 RV308.

# FIG.I
# Restriction Site and Function Map of Plasmid pKC283
# (~9.1 kb)

# FIG.2
# Restriction Site and Function Map of Plasmid pKC283PX
# (~6.1 kb)

# FIG.3
# Restriction Site and Function Map of
# Plasmid pKC283-L
# (~5.9 kb)

# FIG.4
# Restriction Site and Function Map of Plasmid pKC283-LB
## (~5.9 kb)

# FIG.5
# Restriction Site and Function Map of
## Plasmid pKC283PRS
## (~4.0 kb)

# F I G.6

# Restriction Site and Function Map of Plasmid pL32

# (~3.9 kb)

# FIG. 7
# Restriction Site and Function Map of
# Plasmid pNM789
# (~1.0 kb)

# FIG.8
## Construction of Plasmid p120
## (~1.0 kb)

Plasmid pNM789

Pvu II Partial Digestion

Bam HI

Alkaline Phosphatase

Pvu II    Bam HI

Synthetic Fragment

Hind III

lppp

Xba I

Pvu II

ApR

Pvu II
Bam HI

3'lpp

Sal I

T₄ DNA Ligase

Hind III

lppp

Xba I

Pvu II

ApR

Pvu II
Bam HI

3'lpp

Sal I

Plasmid p120

# FIG.9
# Restriction Site and Function Map of
# Plasmid pL47
# (~4.5 kb)

# FIG.IO
# Restriction Site and Function Map of Plasmid pPR12
# (∼5.1 kb)

# FIG.II
# Restriction Site and Function Map of Plasmid pPR12AR1
# (~5.1 kb)

EP 0 361 956 A2

# FIG.12
# Restriction Site and Function Map of Plasmid pL110
# (~6.4 kb)

# FIG.13
## Construction of Plasmid pL110C
## (~6.4 kb)

# FIG.14
# Restriction Site and Function Map of Plasmid pHS246
## (~6.0 kb)